# EUROPÄISCHE PATENTANMELDUNG

(11) **EP 0 631 758 A1**
(43) Veröffentlichungstag der Anmeldung: **04.01.1995**
(21) Anmeldenummer: 94109078.9
(22) Anmeldetag: 14.06.1994
(51) Int. Cl.: A61B 8/06, G01F 1/66

(54) **Implantierbare Messsonde zum Messen der Fliessgeschwindigkeit des Blutes bei Menschen und Tieren**

(30) Priorität: 15.06.1993 DE 4319812
(71) Anmelder: Zurbrügg, Heinz Robert, Dr. med., CH-3047 Bremgarten (CH)
(72) Erfinder: Zurbrügg, Heinz Robert, Dr. med., CH-3047 Bremgarten (CH)
(74) Vertreter: Eitle, Werner, Dipl.-Ing.

(57) **Zusammenfassung**

Eine implantierbare Meßsonde, vorzugsweise Ultraschallsonde zum Messen der Fließgeschwindigkeit des Blutes in natürlichen Blutgefäßen oder Ersatzblutgefäßen von Menschen und Tieren. Die Meßsonde ist mit einem am Ende eines Sondenkabels angeordneten, stabförmigen Sondenkopf (2, 8), in welchem ein Sensor, vorzugsweise ein Piezokristall, eingesetzt ist, und mit einer Einrichtung zum Festhalten des Sondenkopfes am Blutgefäß (7) ausgestattet. Die Abstrahl- und Empfangsfläche (4, 10) des Sensors hat längliche Form, wobei ihre Längsausdehnung mindestens so groß ist wie der Innendurchmesser des Blutgefäßes (7), in dem die Messung durchgeführt werden soll. Mit ihrer Längsausdehnung erstreckt sich diese Fläche (4, 10) im wesentlichen parallel zur Längsachse des stabförmigen Sondenkopfes. Ferner ist diese Fläche zur Festhalteeinrichtung (5, 6) so orientiert, daß sie sich bei am Gefäß befestigten Sondenkopf (2, 8) mit ihrer Längsausdehnung etwa senkrecht zur Blutfließrichtung im Gefäß erstreckt.

## Beschreibung

Die Erfindung betrifft eine implantierbare Meßsonde, vorzugsweise Ultraschallsonde zum Messen der Fließgeschwindigkeit des Blutes in natürlichen Blutgefäßen oder Ersatzblutgefäßen von Menschen und Tieren, mit einem am Ende eines Sondenkabels angeordneten stabförmigen Sondenkopf, in welchem ein Sensor, vorzugsweise ein Piezokristall eingesetzt ist, und mit einer Einrichtung zum Festhalten des Sondenkopfes am Blutgefäß.

Zum Messen der Fließgeschwindigkeit des Blutes sind bereits implantierbare Ultraschallsonden der eingangs genannten Gattung bekannt, die einen Piezokristall von annähernd Kreisform oder quadratischer Form aufweisen (US-A-4 947 854). Da die Abstrahl- und Empfangsfläche des Piezokristalls bei den bekannten Ultraschallsonden im Vergleich zum Innenquerschnitt des Blutgefäßes relativ klein sind und bei Operationen nicht immer sichergestellt werden kann, daß diese Fläche am Sondenkopf so zum Blutgefäß plaziert werden kann, daß mit Sicherheit der für die Messung wesentliche Querschnitt des Blutgefäßes durchstrahlt wird, können mit der bekannten Ultraschallsonde beträchtliche Meßfehler auftreten. Dies ist insbesondere dann der Fall, wenn mit der Ultraschallsonde die im Zentrum des Blutgefäßes gegebenen maximalen Fließgeschwindigkeiten nicht erfaßt werden. Diese Gefahr ist in der Praxis von außerordentlicher Bedeutung. Signaloptimierungsprobleme bei der Implantation der konventionellen Ultraschallsonde können den Chirurgen dazu verleiten, die Sonde zu stark am Gefäß oder dessen Umgebung zu fixieren, was die Gefahr von Verletzungen und/oder sekundären Abknickungen der Blutgefäße erhöht. Zudem sind diese Maßnahmen oftmals mit relevantem Zeitverlust bei der Operation verbunden.

Der Erfindung liegt daher die Aufgabe zugrunde, eine implantierbare Meßsonde der eingangs genannten Gattung zu schaffen, bei der die vom Sensor ausgesandten Strahlen einen so gestalteten Fächer bilden, daß auch bei den in der Regel unvermeidlichen Ungenauigkeiten in der Festlegung der Sonde am Blutgefäß ein optimales Meßergebnis gewährleistet werden kann. Diese Aufgabe wird durch die im kennzeichnenden Teil des Anspruchs 1 angegebene Ausbildung der Meßsonde gelöst. Aufgrund der länglichen Form und der Größe der Längsausdehnung der Abstrahl- und Empfangsfläche des Sensors und dessen im Anspruch 1 angegebenen Anordnung am Blutgefäß, die durch die Festhalteeinrichtung erreicht wird, wird auch bei unvermeidlichen Abweichungen in der Orientierung der Abstrahl- und Empfangsfläche von der Ideallage immer ein optimales Meßergebnis erreicht.

Bei Gefäßen mit einem Innendurchmesser bis zu 3 mm sollte daher die Längsausdehnung der Abstrahl- und Empfangsfläche des Sensors mindestens 3 mm, vorzugsweise mindestens 4 mm sein, bei Gefäßen mit einem Innendurchmesser von 3 bis 6 mm mindestens 3 bis 7 mm, vorzugsweise 9 mm, bei Gefäßen mit einem Innendurchmesser von 3 bis 6 mm mindestens 6 mm, vorzugsweise 9 mm usw. Bei großen Gefäßen mit einem Innendurchmesser von mehr als 20 mm sollte die Längsausdehnung der Abstrahl- und Empfangsfläche des Sensors mehr als 20 mm, vorzugsweise etwa 30 mm sein.

Bei einem bevorzugten Ausführungsbeispiel der erfindungsgemäßen Sonde ist die Längsausdehnung der Abstrahl- und Empfangsfläche ihres Sensors mindestens so groß wie der 1 1/2-fache Innendurchmesser des Blutgefäßes, in dem die Messung durchgeführt werden soll. Zweckmäßig erstreckt sich die Abstrahl- und Empfangsfläche des Sensors im wesentlichen über einen Großteil, mindestens die Hälfte der Längsausdehnung sowie im wesentlichen über die gesamte Querschnittsbreite des stabförmigen Sondenkopfes.

Vorteilhaft besteht die Festhalteeinrichtung aus mindestens einer Klammer, die am Sondenkopf angebracht ist und mit der der Sondenkopf auf das Blutgefäß aufsteckbar ist. Die Klammer kann vom Sondenkopf und mindestens einem Band- oder Drahtstück gebildet sein, das mit seinem einen Ende etwa an oder nahe dem mit dem Sondenkabel verbundenen hinteren Ende des Sondenkopfes befestigt ist und sich an einer Seite des Sondenkopfes im wesentlichen in dessen Längsrichtung erstreckt. Das Band- oder Drahtstück ist zweckmäßig derart federnd biegsam, daß der an einem Blutgefäß festgelegte Sondenkopf durch Ziehen am Sondenkabel in Längsrichtung des Kopfes von dem Blutgefäß abgezogen und ohne operativen Eingriff aus dem Körper des Patienten herausgezogen werden kann. Dabei können sich die Band- oder Drahtstücke an den Sondenkopf heranbiegen oder sich an diesem anlegen.

In der Zeichnung sind zwei besonders zweckmäßige Ausführungsbeispiele der erfindungsgemäßen Ultraschallsonde dargestellt, die im folgenden näher beschrieben werden:
- Fig. 1: zeigt den Sondenkopf des ersten Ausführungsbeispiels in Schrägansicht;
- Fig. 2: zeigt den Sondenkopf gemäß Fig. 1 in Draufsicht;
- Fig. 3: zeigt den Sondenkopf gemäß Fig. 1 in Stirnansicht;
- Fig. 4: zeigt den Sondenkopf des zweiten Ausführungsbeispiels in Schrägansicht;
- Fig. 5: zeigt den Sondenkopf des zweiten Ausführungsbeispiels in Draufsicht, befestigt zwischen einem Blutgefäß und umgebenden Stützgeweben;
- Fig. 6: zeigt einen Schnitt durch den Sondenkopf nach Fig. 4 und 5 und seine Befestigung nach Linie VI-VI in Fig. 5; und
- Fig. 7 bis 9: zeigen die Lage-, Abstrahl- und Empfangsfläche des Sensors gegenüber einem Blutgefäß in verschiedenen, innerhalb der unvermeidlichen Toleranzen.

In der Zeichnung ist mit der Bezugsziffer 1 das Sondenkabel bezeichnet, über welches die Sonde an das in der Zeichnung nicht gezeigte Ultraschallgerät angeschlossen ist, in welchem die Datenerfassung und Auswertung der Messung erfolgt. Am vorderen Ende des Sondenkabels ist der Sondenkopf 2 angeordnet, der stabförmig mit im wesentlichen kreisrunden Querschnitt ausgebildet ist und zweckmäßig aus rostfreiem Stahl besteht. In der Nähe des vorderen Stirnendes 3 dieses Sondenkopfes ist in diesen ein Piezokristall eingesetzt, der eine längliche, ebene oder leicht gekrümmte Abstrahl- und Empfangsfläche 4 hat, die gegenüber dem kreisrunden Umfangsquerschnitt des Sondenkopfes zurückgesetzt ist.

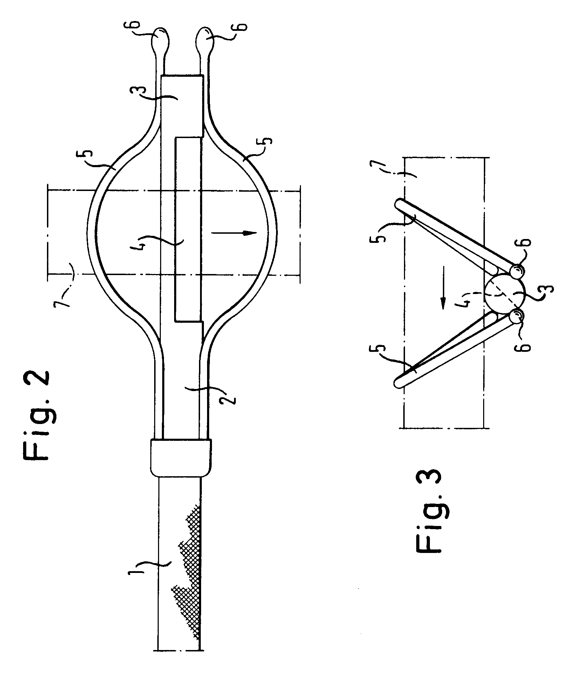

An dem mit dem Sondenkabel 1 verbundenen hinteren Ende des Sondenkopfes 2 sind an zwei gegenüberliegenden Seiten des Kopfes, zu denen sich die Abstrahl- und Empfangsfläche 4 des Piezokristalls in einem Winkel von ca. 45° erstreckt, Klemmdrähte 5 befestigt, die zum Festklemmen des Sondenkopfes an dem Blutgefäß dienen, in welchem die Messung durchgeführt werden soll. Die Klemmdrähte bestehen zweckmäßig aus rostfreiem Federstahl. Ausgehend von ihrem hinteren Ende erstrecken sie sich zunächst neben dem Sondenkopf parallel zu dessen Längsausdehnung, um sich dann im Bereich der Abstrahl- und Empfangsfläche 4 vom Sondenkopf und voneinander wegzubiegen, wobei sich ihr gegenseitiger Abstand wesentlich vergrößert (siehe Fig. 2 und 3). Anschließend sind die Klemmdrähte 5 mit ihren vorderen Enden wieder nahe an den das vordere Ende 3 des Sondenkopfes 2 herangebogen, wobei sie mit diesen Enden etwas über das vordere Stirnende des Sondenkopfes hinausragen. An ihren Enden sind die Klemmdrähte 5 mit Verdickungen 6 versehen, um beim Implantieren des Sondenkopfes übermäßige Beschädigungen im Gewebe zu vermeiden.

Fig. 2 und 3 zeigen, daß der Sondenkopf aufgrund der Anordnung und Ausbildung der Klemmdrähte 5 so auf das Blutgefäß 7, in welchem die Messung stattfinden soll, aufgesteckt und gegenüber diesem ausgerichtet wird, daß die Abstrahl- und Empfangsfläche 4 sich mit seiner Längsausdehnung im wesentlichen senkrecht zur Blutfließrichtung im Blutgefäß 7 und mit ihrer Querausdehnung in einem Winkel von etwa 45° zur Blutfließrichtung erstreckt (Fig. 2 und 3).

Das Ansetzen des Sondenkopfes 2 mit seinen Klemmdrähten 5 an dem Blutgefäß 7 erfolgt dadurch, daß die Klemmdrähte 5 mit ihren vorderen Enden vom vorderen Ende des Sondenkopfes weggebogen werden und gleichzeitig der Sondenkopf in Richtung seiner Längsausdehnung auf das Blutgefäß 7 aufgeschoben wird, wobei die Verdickungen 6 eine Beschädigung des Gewebes verhindern.

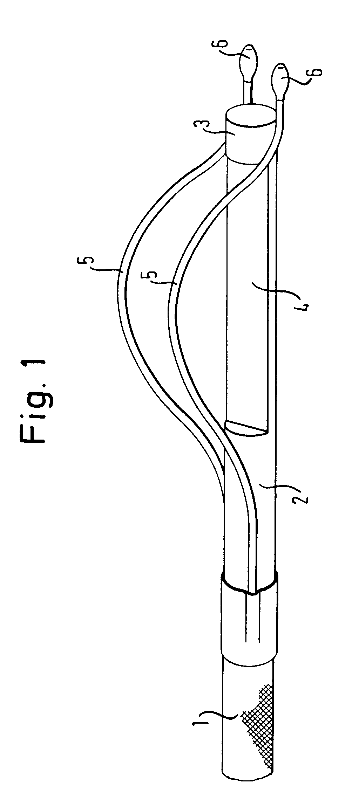

Bei dem in Fig. 1 bis 3 dargestellten Ausführungsbeispiel ist die Längsausdehnung der Abstrahl- und Empfangsfläche 4 des Piezokristalls etwa zweimal so groß wie der Innendurchmesser des Blutgefäßes 7. Dies ist insbesondere bei kleineren Blutgefäßen, also solchen mit einem Außendurchmesser von weniger als 8 mm, zweckmäßig. Bei Blutgefäßen mit größerem Querschnitt kann die Längsausdehnung der Abstrahl- und Empfangsfläche etwas kleiner sein.

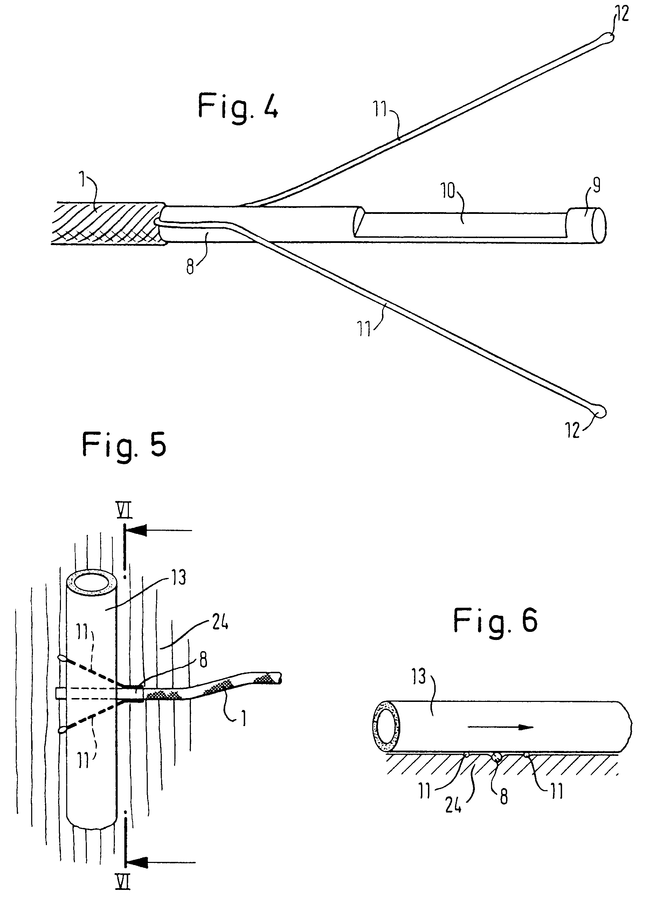

Bei großen Blutgefäßen, z.B. mit einem Außendurchmesser von mehr als 15 mm, wie der Aorta, empfiehlt sich eine Ultraschallsonde mit einem Sondenkopf der in Fig. 4 bis 6 beispielhaft dargestellten Ausführung. Bei diesem Ausführungsbeispiel hat der an das Sondenkabel 1 angesetzte Sondenkopf 8 ebenfalls Stabform, wobei er ebenso wie derjenige des Ausführungsbeispiels gemäß Fig. 1 bis 3 aus rostfreiem Stahl bestehen kann. Auch bei diesem Ausführungsbeispiel ist in der Nähe des vorderen Stirnendes 9 des Sondenkopfes ein Piezokristall mit einer ebenen oder leicht gekrümmten Abstrahl- und Empfangsfläche 10 im Sondenkopf vorgesehen, die gegenüber dem kreisrunden Umfangsquerschnitt des Kopfes zurückgesetzt ist, jedoch zweckmäßig eine größere Längsausdehnung haben kann als die Abstrahl- und Empfangsfläche 4 des ersten Ausführungsbeispiels.

Die an dem hinteren Ende des Sondenkopfes 8 an zwei gegenüberliegenden Seiten des Kopfes befestigten Haltedrähte 11 haben andere Form als die Klemmdrähte 5 beim Ausführungsbeispiel gemäß Fig. 1 bis 3. Während sie wie beim vorgenannten Ausführungsbeispiel zweckmäßig aus rostfreiem Federstahl bestehen können und von ihrem hinteren Ende aus sich zunächst neben dem Sondenkopf parallel zu dessen Längsausdehnung erstrecken, verlaufen sie im Bereich der länglichen Abstrahl- und Empfangsfläche 10 geradlinig schräg zum Sondenkopf auseinander, wobei sie wie die Klemmdrähte des vorgenannten Ausführungsbeispiels an ihren Enden mit Verdickungen 12 versehen sind.

Die so ausgebildeten und geformten Haltedrähte dienen nicht wie die Drähte beim vorgenannten Ausführungsbeispiel als Klammer, mit der der Sondenkopf auf das Blutgefäß aufgesetzt wird, sondern sie sind dazu ausgebildet, den Sondenkopf zwischen dem Blutgefäß 13 und einem benachbarten Stützgewebe 24 des Patienten einzuklemmen und in dieser Lage festzuhalten. Durch ihre Ausrichtung und Lage geschieht dies so, daß die Abstrahl- und Empfangsfläche 10 des Piezokristalls in am Blutgefäß 13 mit Hilfe der Haltedrähte 11 befestigter Lage sich mit ihrer Längsausdehnung im wesentlichen senkrecht zur Blutfließrichtung und mit ihrer Querausdehnung in einem Winkel von etwa 45° zur Blutfließrichtung erstreckt.

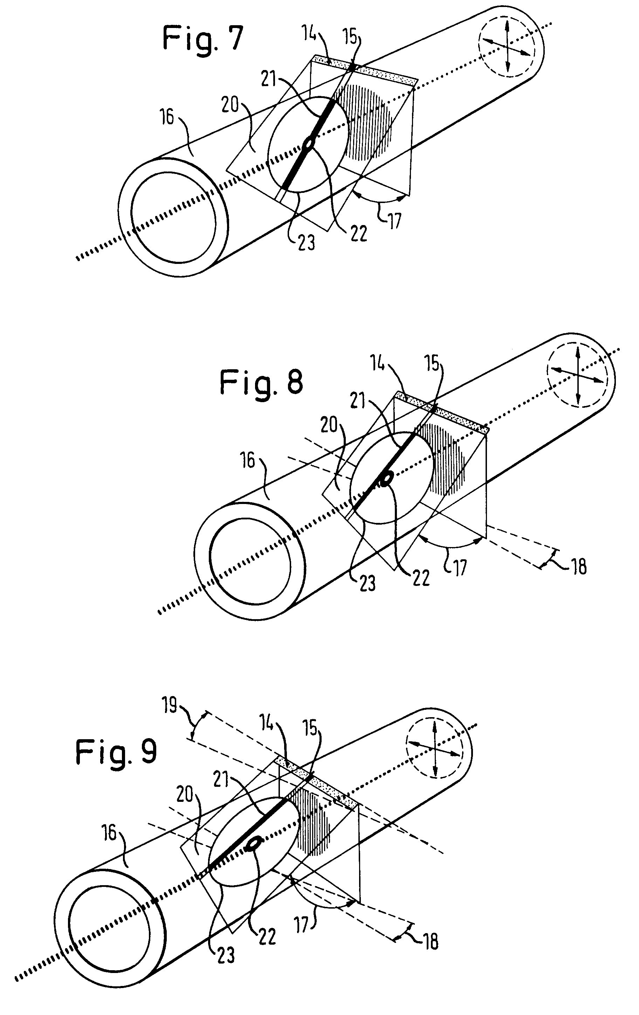

In Fig. 7 bis 9 ist die Wirkung der erfindungsgemäßen Meßsonde einer konventionellen Ultraschallsonde mit relativ kleiner quadratischer oder kreisförmiger Abstrahl- und Empfangsfläche 14 bei unterschiedlich exakter Ausrichtung der Meßsonde gegenüber dem Blutgefäß 16 gezeigt. Die Figuren zeigen, daß die Orientierung der Abstrahl- und Empfangsflächen 14, 15 in beiden Fällen in einer unvermeidlichen Toleranzbreite innerhalb von drei Dimensionen liegen kann.

Fig. 7 zeigt die Orientierung der Abstrahl- und Empfangsfläche in Ideallage ohne jegliche Toleranzabweichung von der Sollage.

Die Neigung der Abstrahlfläche gegenüber der Blutfließrichtung (Winkel 17) beträgt bei dem gezeigten Beispiel idealerweise 45 bis 65°. Eine Abweichung von der Längsausdehnung der Abstrahlfläche zur Senkrechten auf die Blutfließrichtung ist in Fig. 7 in beiden Dimensionen 0°. Fig. 7 zeigt, daß bei diesen genau eingehaltenen Winkeln (Toleranz 0°) kein Unterschied in der Meßgenauigkeit zwischen der erfindungsgemäßen Abstrahlfläche 14 und der konventionellen Abstrahlfläche 15 besteht. Der von der erfindungsgemäßen Abstrahlfläche 14 ausgehende Ultraschallfächer 20 und der von der konventionellen Abstrahlfläche 15 ausgehende Ultraschallstrahl 21 erfassen beide sowohl die schnellsten Blutfließgeschwindigkeiten im Zentrum 22 des Blutgefäßes 16 als auch die langsamsten Blutfließgeschwindigkeiten an der Innenwand 23 des Gefäßes.

Fig. 8 zeigt die Situation beim Auftreten einer kleinen Abweichung der Ausrichtung der Abstrahlflächen 14, 15 in der zweiten Dimension (Toleranzwinkel 18). Hier schneidet der Ultraschallfächer 20 der erfindungsgemäßen Abstrahlfläche 14 wie im Fall von Fig. 7 das Zentrum 22 des Blutgefäßes 16 und registriert somit wie im Fall von Fig. 7 die schnellsten Blutfließgeschwindigkeiten. Dagegen schneidet der Ultraschallstrahl 21 der konventionellen Ultraschallsonde seitlich am Zentrum des Blutgefäßes vorbei, so daß die dort gegebenen schnellsten Blutfließgeschwindigkeiten nicht mehr von der Sonde erfaßt werden. Dagegen werden die langsamsten Blutfließgeschwindigkeiten an der Innenwand des Gefäßes von beiden Abstrahlflächen erfaßt.

Fig. 9 zeigt die Situation bei einer zusätzlichen Toleranzabweichung in der dritten Dimension (Winkel 19). Der Ultraschallstrahl 21 der konventionellen Abstrahlfläche 15 ist gegenüber der Situation von Fig. 8 noch stärker vom Zentrum 22 des Blutgefäßes weggeschwenkt. Da Blutgefäße idealerweise ein parabolisches Flußprofil aufweisen, tritt im Fall von Fig. 9 ein beträchtlicher Meßfehler auf, weil die im Zentrum des Blutgefäßes gegebenen maximalen Fließgeschwindigkeiten nicht erfaßt werden. Beim Ultraschallfächer 20 der erfindungsgemäßen Abstrahlfläche 14 werden dagegen sowohl die zentralen Spitzengeschwindigkeiten als auch die langsameren Geschwindigkeiten an der Gefäßinnenwand 23 voll erfaßt.

## Patentansprüche

1. Implantierbare Meßsonde, vorzugsweise Ultraschallsonde zum Messen der Fließgeschwindigkeit des Blutes in natürlichen Blutgefäßen oder Ersatzblutgefäßen von Menschen und Tieren, mit einem am Ende eines Sondenkabels angeordneten stabförmigen Sondenkopf, in welchem ein Sensor, vorzugsweise ein Piezokristall, eingesetzt ist, und mit einer Einrichtung zum Festhalten des Sondenkopfes am Blutgefäß,
dadurch **gekennzeichnet,** daß die Abstrahl- und Empfangsfläche (4, 10) des Sensors längliche Form hat, wobei ihre Längsausdehnung mindestens so groß ist wie der Innendurchmesser des Blutgefäßes (7), in dem die Messung durchgeführt werden soll, und daß diese Fläche (4, 10) sich mit ihrer Längsausdehnung im wesentlichen parallel zur Längsachse des stabförmigen Sondenkopfes (2, 8) erstreckt und zur Festhalteeinrichtung (5, 11) so orientiert ist, daß sie sich mit ihrer Längsausdehnung etwa senkrecht zur Blutfließrichtung im Gefäß (7, 13) erstreckt.

2. Sonde nach Anspruch 1, dadurch gekennzeichnet, daß die ausgesandten Schallwellen einen Fächer bilden, der bei am Gefäß befestigten Sondenkopf (2, 8) den gesamten oder annähernd gesamten Fließquerschnitt des Blutgefäßes (7, 13) durchsetzt.

3. Sonde nach Anspruch 1, dadurch gekennzeichnet, daß die Längsausdehnung der Abstrahl- und Empfangsfläche (4, 10) mindestens so groß ist wie der 1 1/2-fache Innendurchmesser des Blutgefäßes (7).

4. Sonde nach Anspruch 1, dadurch gekennzeichnet, daß die Länge der Abstrahl- und Empfangsfläche (4, 10) mindestens so groß ist wie die 1 1/2-fache Breite dieser Fläche.

5. Sonde nach Anspruch 1, dadurch gekennzeichnet, daß die Längsausdehnung der Abstrahl- und Empfangsfläche (4, 10) mindestens so groß wie die 2 1/2-fache Breite, vorzugsweise die 3 1/2-fache Breite, dieser Fläche ist.

6. Sonde nach Anspruch 1, dadurch gekennzeichnet, daß die Abstrahl- und Empfangsfläche (4, 10) zur Festhalteeinrichtung (5, 11) so orientiert ist, daß sie sich bei am Gefäß befestigtem Sondenkopf (2, 8) mit ihrer Querausdehnung in einem Winkel von 0° bis 90° zur Blutfließrichtung erstreckt.

7. Sonde nach Anspruch 1, dadurch gekennzeichnet, daß die Abstrahl- und Empfangsfläche (4, 10) zur Festhalteeinrichtung (5, 11) so orientiert ist, daß sie sich bei am Blutgefäß befestigtem Sondenkopf (2, 8) mit ihrer Querausdehnung in einem Winkel, je nach Gefäßgröße, von etwa 30° bis 60° zur Blutfließrichtung erstreckt.

8. Sonde nach Anspruch 1, dadurch gekennzeichnet, daß die Abstrahl- und Empfangsfläche (4, 10) der Sonde von mehreren Sensoren gebildet ist.

9. Sonde nach Anspruch 1, dadurch gekennzeichnet, daß die Abstrahl- und Empfangsfläche (4, 10) des Sensors unmittelbar vor dem vom Sondenkabel (1) abgewandten vorderen Ende (3, 9) des stabförmigen Sondenkopfes (2, 8) an dessen Längsseite angeordnet ist.

10. Sonde nach Anspruch 1, dadurch gekennzeichnet, daß sich die Abstrahl- und Empfangsfläche (4, 10) des Sensors im wesentlichen über die gesamte Querschnittsbreite des stabförmigen Sondenkopfes (2, 8) erstreckt.

11. Sonde nach Anspruch 1, dadurch gekennzeichnet, daß die Festhalteeinrichtung (5) aus mindestens einer Klammer besteht, die am Sondenkopf (2, 8) angebracht ist, und mit der der Sondenkopf an das Blutgefäß (7) anklemmbar ist.

12. Sonde nach Anspruch 11, dadurch gekennzeichnet, daß die Klammer vom Sondenkopf (2, 8) und mindestens einem Band- oder Drahtstück (5) gebildet ist, das mit seinem einen Ende etwa an oder nahe dem mit dem Sondenkabel (1) verbundenen hinteren Ende des stabförmigen Sondenkopfes (2, 8) befestigt ist und sich an einer Seite des Sondenkopfes im wesentlichen in dessen Längsrichtung erstreckt.

13. Sonde nach Anspruch 12, dadurch gekennzeichnet, daß das Band- oder Drahtstück (5) mit seinem anderen freien Ende nahe oder unmittelbar neben dem vorderen, vom Sondenkabel (1) abgewandten Ende des Sondenkopfes (2, 8) enden.

14. Sonde nach Anspruch 12, dadurch gekennzeichnet, daß das Band- oder Drahtstück (5) mit seinem anderen vorderen Ende über das vom Sondenkabel (1) abgewandte vordere Ende (3) des Sondenkopfes (2, 8) hinausragt.

15. Sonde nach einem oder mehreren der Ansprüche 12 bis 14, dadurch gekennzeichnet, daß das Band- oder Drahtstück (5) im Bereich der Abstrahl- und Empfangsfläche (4) des Sensors bauchförmig vom Sondenkopf (2, 8) weggebogen ist.

16. Sonde nach Anspruch 12, dadurch gekennzeichnet, daß das Band- oder Drahtstück (5) derart federnd biegsam ist, daß der an einem Blutgefäß (7, 13) festgelegte Sondenkopf (2, 8) durch Ziehen am Sondenkabel (1) in Längsrichtung des Sondenkopfes (2, 8) von dem Blutgefäß abziehbar ist.

17. Sonde nach den Ansprüchen 11 und 12, dadurch gekennzeichnet, daß die Klammer mindestens zwei aus Drahtstücken bestehende Klemmdrähte (5) aufweist, die an je einer Seite des Sondenkopfes befestigt sind.

18. Sonde nach Anspruch 17, dadurch gekennzeichnet, daß die Klemmdrähte (5) im Bereich der Abstrahl- und Empfangsfläche (4) des Sensors bauchförmig vom Sondenkopf (2, 8) weggebogen sind.

19. Sonde nach Anspruch 18, dadurch gekennzeichnet, daß der gegenseitige Abstand der Klemmdrähte (5) in ihrem ausgebauchten Längenbereich wesentlich größer ist als an ihren beiden Enden.

20. Sonde nach Anspruch 11, dadurch gekennzeichnet, daß die Festhalteeinrichtung mindestens zwei Haltedrähte (11) aufweist, die an je einer Seite des Sondenkopfes befestigt sind und sich zu ihrem vorderen Ende hin im wesentlichen geradlinig voneinander und vom Sondenkopf (8) entfernen.

21. Sonde nach einem oder mehreren der Ansprüche 11 bis 20, dadurch gekennzeichnet, daß die freien Enden des oder der Band- oder Drahtstücke (5, 11) abgerundet sind.

22. Sonde nach einem oder mehreren der Ansprüche 11 bis 20, dadurch gekennzeichnet, daß die freien Enden des oder der Band- oder Drahtstücke (5, 11) mit runden Verdickungen (6, 12) versehen sind.
